# EUROPEAN PATENT APPLICATION

(11) **EP 1 236 706 A1**
(43) Date of publication of application: **04.09.2002**
(21) Application number: 00970026.1
(22) Date of filing: 24.10.2000
(51) Int. Cl.: C05D 1/02, C07C 31/38

(54) **POTASSIUM CHLORIDE FOR FERTILIZER AND PROCESS FOR PRODUCING THE SAME**

(30) Priority: 29.10.1999 JP 30890599
(71) Applicant: F-TECH, INC., Chuo-ku, Tokyo 104-0031 (JP)
(72) Inventor: NUKUI, Kazunori, Tokuyama-shi, Yamaguchi 745-0005 (JP); ABE, Hiroyuki, Hofu-shi, Yamaguchi 747-0023 (JP); NISHIDA, Takuji, Shinnanyo-shi, Yamaguchi 746-0011 (JP); ARAI, Shoji, Tokuyama-shi, Yamaguchi 745-0816 (JP)
(74) Representative: Ratcliffe, Susan Margaret
(86) International application number: JP0007419
(87) International publication number: WO01032585

(57) **Abstract**

By making use of potassium chloride by-product that is produced in large amounts in a production process of 2,2,2-trifluoroethanol and its esters, potassium chloride suitable for use as a fertilizer is produced. Accordingly, the present invention makes it possible not only to effectively make use of the potassium chloride by-product, but also to reduce the cost in processing the waste products and prevent environmental pollution.

The present invention provides a potassium chloride by-product for use as a fertilizer, the potassium chloride by-product produced in a process in which 1,1,1-trifluoro-2-chloroethane is reacted with a potassium salt of an organic carboxylic acid in the presence of polar aprotic solvent to form 2,2,2-trifluoroethanol and/or 2,2,2-trifluoroethyl ester of organic carboxylic acid, the potassium chloride by-product being characterized in that it contains organic materials in an amount of 3wt% or less.

## Description

### TECHNICAL FIELD

The present invention relates to a potassium chloride by-product for use as a fertilizer produced in a specific reaction system and a process for producing the same.

### BACKGROUND ART

Potassium components are essential for the growth of plants, as are phosphorus components and nitrogen components. For this reason, potassium salts are used as the material for a potash fertilizer or the material for a balanced fertilizer in which potassium salt is blended with ammonium phosphate, triple superphosphate, calcined superphosphate, and ammonium sulfate. Potassium chloride is commonly in use as the potassium component and is generally made from mine ores containing inorganic potassium salts.

Caustic potassium and other potassium compounds are often used in the synthesis of useful organic compounds. In general, these compounds, after being exploited, are converted to potassium salts, which in most cases are discarded as industrial wastes because of a limitation on the amount and for economical reasons.

2,2,2-trifluoroethanol and its esters are industrially useful compounds and are used in various applications including a material for medicines, heating medium and a material for various polymers. These compounds are produced by reacting 1,1,1-trifluoro-2-chloroethane with a potassium salt of organic carboxylic acid in the presence of polar aprotic solvent. Such a process produces potassium chloride by-product in large amounts. For example, processes disclosed in Japanese Patent Publication No. Sho 64-9299, Japanese Patent Publication No. Sho 64-9300, and Japanese Patent Publication No. Hei 2-9569 involve use of ã-butyrolactone as a solvent and potassium acetate or potassium ã-hydroxybutyrate as a reactant. Also, International Patent Publication No. WO97/21656 and Japanese Patent Laid-Open Publication No. Sho 56-13742 disclose processes that involve use of 1,3-dimethyl-2-imidazolidinone, N-methyl-2-pyrrolidone. sulfolane, dimethyl sulfoxide, dimethyl formamide, or dimethyl acetamide as a solvent and involve reaction of 1,1,1-trifluoro-2-chloroethane with potassium acetate or potassium 6-hydroxyhexanoate. Any of these processes produces potassium chloride by-product in large amounts.

The large amount of potassium chloride produced as a by-product in the above-described processes (which is referred to as potassium chloride by-product, hereinafter) is an important natural resource, and a way to make efficient use of these by-products has been hoped for. The potassium chloride by-products, however, are known to inhibit budding or the growth of plants though the underlying mechanisms have not been made clear. For this reason, their use as a source of potassium for fertilizers has been deemed unviable. On the other hand, attempts to process the potassium chloride by-products as an industrial waste not only provoke concerns about their effects on a landfill or surrounding environments, but also require enormous costs, making the process industrially impractical. Similarly, attempts to dispose of the potassium chloride by-products by dissolving them in water have failed since the resulting aqueous solution, which contains organic materials in abundance, has extremely high COD and BOD values and requires a dedicated effluent-processing facility. Requiring large scale processing facilities and enormous costs, this approach has also posed serious problems.

### DISCLOSURE OF THE INVENTION

Accordingly, it is an objective of the present invention to make efficient use of potassium chloride by-products, which are produced in large amounts in the production process of 2,2,2-trifluoroethanol and its esters, by making them suitable for use as a fertilizer and to thereby reduce the costs associated with disposal of the by-products and prevent environmental pollution.

In the course of their studies on potassium chloride by-products, the present inventors have found that a potassium chloride by-product does not inhibit budding or the growth of plants and thus can be used as a material of fertilizers if the amount of organic materials present in the potassium chloride by-product is maintained at a predetermined amount or less. This finding ultimately led the present inventors to complete the present invention.

Thus, one aspect of the present invention provides potassium chloride for use as a fertilizer. This potassium chloride is produced as a by-product in a process for producing 2,2,2-trifluoroethanol and/or a 2,2,2-trifluoroethyl ester of an organic carboxylic acid in which 1,1,1-trifluoro-2-chloroethane is reacted with a potassium salt of organic carboxylic acid in the presence of a polar aprotic solvent. The potassium chloride is characterized in that it contains organic compounds in an amount of 3wt% or less.

In another aspect, the present invention provides a process for producing potassium chloride for use as a fertilizer. This potassium chloride is produced as a by-product in a process for producing 2,2,2-trifluoroethanol and/or 2,2,2-trifluoroethyl ester of an organic carboxylic acid in which 1,1,1-trifluoro-2-chloroethane is reacted with a potassium salt of organic carboxylic acid in the presence of a polar aprotic solvent. The process is characterized in that the potassium chloride by-product is purified.

In a further aspect, the present invention provides a balanced fertilizer composed of the potassium chloride obtained in accordance with the present invention and other fertilizers.

Potassium chloride in accordance with the present invention for use as a fertilizer is produced as a by-product in a process for producing 2,2,2-trifluoroethanol and/or 2,2,2-trifluoroethyl ester of an organic carboxylic acid in which 1,1,1-trifluoro-2-chloroethane is reacted with a potassium salt of organic carboxylic acid in the presence of a polar aprotic solvent. The potassium chloride is characterized in that it contains organic compounds in an amount of 3wt% or less.

Examples of the polar aprotic solvent include ã-butyrolactone, 1,3-dimethyl-2-imidazolidinone, N-methyl-2-pyrrolidone, sulfolane, dimethyl sulfoxide, dimethyl formamide, and dimethyl acetamide. Of these, ã-butyrolactone, 1,3-dimethyl-2-imidazolidinone, N-methyl-2-pyrrolidone, and sulfolane are particularly preferred.

1,1,1-trifluoro-2-chloroethane serving as the material may be produced in a known process in which, for example, trichloroethylene is reacted with hydrofluoric acid in the presence of a catalyst. Though not limited to a particular purity, the purity of 1,1,1-trifluoro-2-chloroethane is preferably 98% or higher. It may well contain impurities, such as 1,1,1,2-tetrafluoroethane (HFCl34a), that are not involved in the present reaction.

Examples of the potassium salt of organic carboxylic acid include potassium salts of saturated or unsaturated aliphatic monocarboxylic acids such as formic acid, acetic acid, propionic acid, butyric acid, acrylic acid, methacrylic acid, crotonic acid, oxalic acid, malonic acid, maleic acid, succinic acid, adipic acid, ã-hydroxybutyric acid, and 6-hydroxyhexanoic acid; potassium salts of aliphatic dicarboxylic acids and aliphatic carboxylic acids having a hydroxyl group; and potassium salts of aromatic carboxylic acids such as benzoic acid, phenylacetic acid, salicylic acid, and phthalic acid. These potassium salts of organic carboxylic acids can readily be synthesized from commercially available organic carboxylic acids and caustic potassium, or commercial products may be used if available.

According to the present invention, the above-described materials may be used to carry out the reaction for producing 2,2,2-trifluoroethanol and/or 2,2,2-trifluoroethyl ester of an organic carboxylic acid, in which potassium chloride by-products are produced. Processes and conditions of the reaction may be properly selected according to the aforementioned literatures. As a typical example, a process is described in the following, in which ã-butyrolactone is used as a solvent and 1,1,1-trifluoro-2-chloroethane is reacted with potassium ã-hydroxybutyrate to synthesize 2,2,2-trifluoroethanol. First, ã-butyrolactone is placed in a pressurized reactor, and 1,1,1-trifluoro-2-chloroethane and potassium ã-hydroxybutyrate are added. Then, the reaction is allowed to proceed for 4 to 8 hours at a reaction temperature of 180 C to 220 C while stirring the reaction mixture to complete the reaction. Unreacted 1,1,1-trifluoro-2-chloroethane is then removed, and the reaction mixture in the pressurized container is transferred to a distillation column, where it is subjected to crude distillation to distill out 2,2,2-trifluoroethanol and water. The solution remaining in the container contains the potassium chloride by-product in the form of slurry, as well as ã-butyrolactone and heavy fraction.

The potassium chloride by-product is collected from the slurry by separating from ã-butyrolactone and the heavy fraction through processes such as filtration and centrifugation. The remaining solution is again used in the reaction whereas the separated potassium chloride by-product, which still contains solvent, is heated under reduced pressure to collect the solvent.

The potassium chloride by-product so obtained is a white or faintly brownish solid and is composed of particles sized several to several hundred microns. This potassium chloride by-product generally contains little water while the water content may vary depending on the type of the solvent and conditions for the reaction, and without proper processing, it cannot be used as a potassium source for fertilizer. In an effort to make use of the potassium chloride by-products, the present inventors have unexpectedly found that if it contains organic materials in an amount of 3wt% or less, the potassium chloride by-product can be used as a potassium source for fertilizers without causing any problems.

The amount of organic materials can be easily determined by, for example, heating the potassium chloride by-product in air at 600 C for 1 hour in an electric furnace and measuring the decrease in weight.

It has been demonstrated in the juvenile plant tests conducted according to *59 NOSAN* No. 1943 (The ministry of Agriculture, Forestry and Fisheries of Japan. 1984) that the potassium chloride by-products other than those specially treated to reduce the amount of organic materials to 3wt% or less, that is, the potassium chloride by-products containing more than 3wt% of the organic materials inhibit budding and growth of plants. Although underlying mechanisms are not clear yet, it is believed that the organic materials present in the potassium chloride by-products. especially those containing fluorine, affect budding of plants as well as the growth of the plants that follows budding.

While the potassium chloride by-products of the present invention containing 3wt% or less of organic materials can be used as the potassium chloride for use in fertilizer without further processing, those containing more than 3wt% of organic materials may also be used by purifying the potassium chloride by-products to reduce the amount of organic materials to 3wt% or less. Purification is preferably carried out, for example, by washing with a solvent or burning, although other means are also contemplated.

The burning process of the present invention for purifying the potassium chloride by-products includes a batch process in which the by-products are burned by flame on a proper pan, and a continuous process based on the counter flow technique, in which the by-products are burned, for example, in a rotary kiln to which flame is applied from the bottom discharge port thereof and the potassium chloride by-products are continuously supplied from above. Any proper processes can be used to burn the potassium chloride by-products. The burning process is carried out at a temperature of 300 C or higher, preferably at 400 C or higher. If the burning temperature is lower than 300 C, organic materials present in the potassium chloride by-products remain unburned and the aforementioned objective will not be achieved. The burning process is typically carried out over a time period of 30min to 1 hour though the time may be properly selected depending on equipment used and burning conditions. If the time is shorter than the specified period, organic materials remain unburned and the aforementioned objective will not be achieved.

In the present invention, the washing process for washing the potassium chloride by-products with an organic solvent for the purpose of purification includes a batch process, in which a slurry formed by the potassium chloride by-products and an organic solvent placed in the same container is stirred to wash the potassium chloride by-products. a continuous process, and a rinse process, in which an organic solvent is directly sprayed onto the potassium chloride by-products for washing.

Organic solvents that can be used to wash the potassium chloride by-products include alcohols, ketones, esters, ethers, amines, hydrocarbons, halogenated hydrocarbons, and aromatic compounds. Of these, alcohols, ketones, and esters are preferred since these solvents can be used in small amounts and the solvents need to be used a fewer number of times to complete washing. Also, these solvents are easy to handle, inexpensive, easy to collect, and inflict less damage to the environment. Although ketones such as acetone, methyl ethyl ketone, methyl *t*-butyl ketone and cyclohexanone, and esters such as ethyl acetate ester and butyl acetate ester are preferably used, alcohols are the most preferred solvents. Examples of the alcohol include aliphatic or aromatic alcohols having 8 carbons or less and polyols having two or more hydroxyl groups. Specific examples include methanol, ethanol, propanol, isopropanol, butanols, pentanols, hexanols, octanols, cyclopentanol, cyclohexanol, cyclohexylmethanol, benzylalcohol, ethyleneglycol, diethyleneglycol, polyethyleneglycol monomethyl ethers, propyleneglycol, polyalkyleneglycol monoalkyl ethers, butanediols, hexanediols, and glycerol. Of these, methanol, ethanol, propanol, isopropanol, butanols, and mixtures thereof are particularly preferred with methanol being most preferred.

Methanol, the most preferred solvent, exhibits an enhanced ability to wash the potassium chloride by-products if it contains water. The amount of water is from 1 to 50wt%, preferably 5 to 20wt%. If the water content is less than 1wt%, the washing ability is decreased whereas the amount of the potassium chloride by-products that can dissolve in the alcohol for washing is increased and the by-products will be circulated if the water content exceeds 50wt%. This is economically disadvantageous.

With regard to the washing process using the slurry of the potassium chloride by-products, the slurry preferably contains the potassium chloride by-products at a concentration of 10 to 70wt% although the concentration is not limited to the specified range. If this concentration is less than 10wt%, the amount of the solvent required becomes too large, which is economically unfavorable, whereas the concentration exceeding 70wt% not only reduces the washing ability but also makes it difficult to mix and stir the slurry. The slurry preferably has a temperature of 0 to 100 C, preferably 20 to 60 C although the temperature is not limited to the specified ranges. The washing ability is reduced at temperatures lower than 0 C whereas dangerous alcohol vapor is produced at temperatures higher than 100 C. The washing process is typically carried out over a time period of 0.1 to 1 hour. Washing is insufficient if the washing time is too short while a corresponding increase in the washing ability is not expected if the washing time is too long.

After washing is finished, the slurry is separated by filtration, decantation or other techniques. When necessary, the washing process may be repeated using alcohol. Subsequently, water and alcohol are removed through a drying process to obtain the potassium chloride for use as fertilizer. The alcohol used in the washing process can be collected for recycling through processes such as distillation.

As set forth, the potassium chloride for use as fertilizer is obtained through purification by washing with a solvent or burning according to the present invention. The potassium chloride for fertilizer must contain organic materials in an amount of 3wt% or less. If the amount of organic materials contained in the potassium chloride for fertilizer exceeds 3wt%, budding and growth of plants are affected, making the use of the potassium chloride as a fertilizer difficult.

The potassium chloride for use as fertilizer may be used as a balanced fertilizer by blending with phosphorus- or nitrogen-containing fertilizers such as ammonium sulfate, triple superphosphate, calcined superphosphate, and ammonium sulfate.

### BEST MODE FOR CARRYING OUT THE INVENTION

The present invention will now be described in further detail in reference to Examples, which are provided by way of example only and do not limit the scope of the invention in any way.

### Reference Example 1

11.0kg ã-butyrolactone, 0.36kg water, 2.17kg potassium ã-hydroxybutyrate, and 2.37kg 1,1,1-trifluoro-2-chloroethane were placed in a 20L autoclave equipped with a magnetic stirrer. The autoclave was sealed off and heated to 200 C and the reaction was allowed to proceed for 6 hours. After the reaction period, 1,1,1-trifluoro-2-chloroethane, 2,2,2-trifluoroethanol, and ã-butyrolactone, which was accompanied by water, were extracted through simple distillation. The residues were allowed to cool to room temperature, and then the potassium chloride by-products were filtered out. The obtained solid products were dried at 110 C for 4 hours under 2.0kPa to obtain 1.50kg raw salt.

The raw salt contained no water and 8.2wt% of organic materials. The amount of organic materials was determined by heating the raw salt in air at 600 C for 1 hour and measuring the decrease in weight.

### Reference Example 2

10kg N-methylpyrrolidone, 2.48kg potassium methacrylate, and 2.84kg 1,1,1-trifluoro-2-chloroethane were placed in a 20L autoclave equipped with a magnetic stirrer. The autoclave was sealed off and heated to 180 C and the reaction was allowed to proceed for 6 hours.

After the reaction period, 1,1,1-trifluoro-2-chloroethane, 2,2,2-trifluoroethylmethacrylate, and N-methylpyrrolidone were extracted through simple distillation. The residues were allowed to cool to room temperature, and then the potassium chloride by-products were filtered out. The obtained solid products were dried at 110 C for 4 hours under 2.0kPa to obtain 1.50kg raw salt.

The raw salt contained no water and 5.5% of organic materials. The amount of organic materials was determined by heating the raw salt in air at 600 C for 1 hour and measuring the decrease in weight.

### Example 1

50g of the raw salt obtained in Reference Example 1 were mixed with 50g methanol containing 10wt% of water, and the mixture was stirred for 30min. Following this, the potassium chloride was filtered out through vacuum filtration and was rinsed with 10g methanol containing 10wt% of water. The resulting potassium chloride was dried in a desiccator at 110 C for 1 hour. The amount of organic materials in the resulting potassium chloride for fertilizer was measured to be 0.5wt%.

### Examples 2 to 12 and Comparative Example 1

Products were obtained in the same manner as in Example 1 using different solvents in different amounts while water contents were varied. The results are shown in Table 1.

**Table 1**

| | Raw salt | Organic solvent for washing | | | Potassium chloride for fertilizer | |
|---|---|---|---|---|---|---|
| | | Type | Weight(g) | Water content (wt%) | Weight (g) | Organic material content (wt%) |
| Comp.Ex.1 | 50 | Methanol | 20 | 0 | 47 | 4.0 |
| Ex.2 | 50 | Methanol | 25 | 10 | 45 | 0.9 |
| Ex.3 | 50 | Methanol | 100 | 10 | 42 | 0.4 |
| Ex.4 | 50 | Methanol | 50 | 3 | 47 | 2.2 |
| Ex.5 | 50 | Methanol | 50 | 5 | 42 | 1.5 |
| Ex.6 | 50 | Methanol | 50 | 20 | 42 | 0.7 |
| Ex.7 | 50 | Methanol | 50 | 40 | 42 | 1.9 |
| Ex.8 | 50 | Ethanol | 100 | 10 | 45 | 1.8 |
| Ex.9 | 50 | Isopropyl alcohol | 100 | 10 | 45 | 2.5 |
| Ex.10 | 50 | Acetone | 50 | 10 | 45 | 2.8 |
| Ex.11* | 50 | Acetone | 50 | 10 | 45 | 0.9 |
| Ex.12 | 50 | Ethyl acetate | 150 | 0 | 45 | 2.1 |

| | | | | | | |
|---|---|---|---|---|---|---|
| * Washing is repeated three times. | | | | | | |

### Example 13

50g raw salt was spread on a ceramic pan and was burned in air at 400 C for 1 hour using an electric furnace. The amount of organic materials in the resulting potassium chloride for fertilizer was measured to be 0.1wt%.

### Example 14

50g of the raw salt obtained in Reference Example 2 were mixed with 50g methanol containing 10wt% of water, and the mixture was stirred for 30min. Following this, the potassium chloride was filtered out through vacuum filtration and was rinsed with 10g methanol containing 10wt% of water. The resulting potassium chloride was dried in a desiccator at 110 C for 1 hour. The amount of organic materials in the resulting potassium chloride for fertilizer was measured to be 0.3wt%.

### Examples 15 through 19 and Comparative Examples 2 through 4

According to *59 NOSAN* No. 1943 (The ministry of Agriculture, Forestry and Fisheries of Japan. 1984), the juvenile plant budding/growth tests were conducted using surface humic andosol as a sample soil and Komatsuna plant(*Brassica rapa* var. *pervidis* or var. *komatsuna)* as a sample crop. The potassium chloride by-products of the present invention were used as sample fertilizers while a potassium chloride reagent (special grade reagent with the purity of 95% or higher, manufactured by Kanto Kagaku Co. Ltd.,) was used as a control fertilizer. A set of pots each containing 0.16g fertilizer were prepared for each fertilizer to serve as a standard fertilized section. Fertilized sections with planters containing twice, three times, and four times the standard amount were also prepared for each fertilizer. Each pot was planted with 20 seeds of Komatsuna plant, and budding and the growth of the plants were observed. Budding and the growth observed for different amounts of organic materials in the potassium chloride by-products are as shown in Table. 2 below.

**Table. 2**

| | Organic material content in potassium chloride by-products (wt%) | Budding and growth of plants |
|---|---|---|
| Ex.15 | 2.8 | No significant difference from the control section. |
| | (purified salt of Ex.10) | |
| Ex.16 | 0.4 | No significant difference from the control section. |
| | (purified salt of Ex.3) | |
| Ex.17 | 2.5 | No significant difference from the control section. |
| | (purified salt of Ex.9) | |
| Ex.18 | 0.1 | No significant difference from the control section. |
| | (purified salt of Ex.13) | |
| Ex.19 | 0.3 | No significant difference from the control section. |
| | (purified salt of Ex.14) | |
| Comp.Ex.2 | 4.0 | No significant difference observed in budding. Growth reduced in plants in pots containing a larger amount of the fertilizer. |
| | (purified salt of Comp.Ex.1) | |
| Comp.Ex.3 | 8.2 | Budding and growth reduced in plants in pots containing a larger amount of the fertilizer. |
| | (purified salt of Ref.Ex.1) | |
| Comp.Ex.4 | 5.5 | Budding and growth reduced in plants in pots containing a larger amount of the fertilizer. |
| | (purified salt of Ref.Ex.2) | |

### Example 20

Calcium superphosphate (CaH₄(PO₄)₂ H₂O) was mixed with the potassium chloride for fertilizer obtained in Example 10 and water at a ratio of 1:1:1 to form a slurry. The slurry was dried and granulated to form a balanced fertilizer. Using this balanced fertilizer as a sample fertilizer and another balanced fertilizer as a control fertilizer, which was prepared in the same manner as the first balanced fertilizer using the reagent potassium chloride, the juvenile plant budding/growth tests were conducted in the same manner as in Example 14. No significant difference was observed in budding and growth of the plant between the sample and the control.

### INDUSTRIAL APPLICABILITY

Potassium chloride by-products produced in processes in which 1,1,1-trifluoro-2-chloroethane is reacted with a potassium salt of an organic carboxylic acid in the presence of polar aprotic solvent to form 2,2,2-trifluoroethanol and/or an 2,2,2-trifluoroethyl ester of organic carboxylic acid poses a problem since it is difficult to discard such by-products in the form of a solution and processing such by-products requires a large scale facility and a significant cost. These potassium chloride by-products inhibit budding and the growth of plants due to substantial amounts of organic materials they contain and are thus not suited for use as a material of fertilizer.

However, the potassium chloride by-products in accordance with the present invention, which contains, or is processed to contain, organic materials in an amount of 3wt% or less, finds its application as a fertilizer. The organic materials present in the potassium chloride by-products are effectively removed by burning or washing with an organic solvent. Accordingly, the present invention is of considerable significance in terms of reduction of the costs required to process the potassium chloride by-products, recycling of the by-products, and suppression of damage to the environment.

## Claims

1. A potassium chloride by-product for use as a fertilizer, the potassium chloride by-product produced in a process in which 1,1,1-trifluoro-2-chloroethane is reacted with a potassium salt of an organic carboxylic acid in the presence of polar aprotic solvent to form 2,2,2-trifluoroethanol and/or 2,2,2-trifluoroethyl ester of organic carboxylic acid, the potassium chloride by-product being **characterized in that** it contains organic materials in an amount of 3wt% or less.

2. A process for producing potassium chloride for use as a fertilizer, the process **characterized in that** purifying a potassium chloride by-product produced in a process in which 1,1,1-trifluoro-2-chloroethane is reacted with a potassium salt of an organic carboxylic acid in the presence of polar aprotic solvent to form 2,2,2-trifluoroethanol and/or 2,2,2-trifluoroethyl ester of organic carboxylic acid to reduce the amount of organic materials contained in the potassium chloride by-product to 3wt% or less.

3. The process for producing potassium chloride for use as a fertilizer according to claim 1, **characterized in that** the purification is done by burning or washing with an organic solvent.

4. The process for producing potassium chloride for use as a fertilizer according to claim 3, **characterized in that** the organic solvent is at least one selected from the group consisting of alcohols, ketones, and esters.

5. The process for producing potassium chloride for use as a fertilizer according to claim 3 or 4, **characterized in that** the organic solvent is an alcohol.

6. The process for producing potassium chloride for use as a fertilizer according to any one of claims 3 to 5, **characterized in that** the alcohol is methanol.

7. The process for producing potassium chloride for use as a fertilizer according to any one of claims 3 to 6, **characterized in that** the methanol contains water in an amount of 1 to 50wt%.

8. A balanced fertilizer **characterized in that** it is composed of the potassium chloride according to claim 1 and other fertilizers.

9. The balanced fertilizer according to claim 8 **characterized in that** the other fertilizers contain a phosphate component and/or a nitrogen component.
